# EUROPEAN PATENT APPLICATION

(11) **EP 2 353 384 A2**
(43) Date of publication of application: **10.08.2011**
(21) Application number: 10192066.8
(22) Date of filing: 22.11.2010
(51) Int. Cl.: A01N 25/02, A01N 25/22, A01N 35/02, A01P 1/00, A01N 35/08, A01N 37/34

(54) **Disinfectant formulations that remain liquid at low temperature**

(30) Priority: 18.12.2009 EP 09425511
(71) Applicant: Dow Global Technologies LLC, Midland, MI 48674 (US); Dow Italia Divisione Commerciale S.r.l., 20151 Milano (IT)
(72) Inventor: Arzu, Antonio, 25037 Pontoglia (IT); Hughes, Stephanie, Lynn, Beaverton, MI 48612 (US); Bertheas, Ute, Helmine, 8805 Richterswil (CH); Keene, Philip, Alexander, 8810 Horgen (CH); Foley, Paul, Midland, MI 48642 (US); Lenoir, Pierre, Marie, 8805 Richterswil (CH)
(74) Representative: Buckley, Guy Julian

(57) **Abstract**

In the present invention, there is provided a disinfectant composition comprising
(a) 15% to 49.75% biocide, by weight based on the weight of said composition,
(b) 15% to 49.75% water, by weight based on the weight of said composition, and
(c) 0.5% to 60% soluble salt, by weight based on the weight of said composition.

## Description

### BACKGROUND:

It is often desired to use liquid formulations that contain biocides. In some situations, it is desired to use such liquid formulations at low temperature, and then it is desired that the formulation remain in the liquid state. It is also desirable to minimize the amount of solvent that is used in the formulation. In some cases, it is also desirable that the formulation have one or more of the following characteristics: chemical stability over a reasonable period of time, without undergoing undesirable chemical reactions such as, for example, degradation of the biocide; relatively high flash point; relatively low human toxicity; relatively low tendency to promote corrosion; and relatively high concentration of biocide.

US 5,496,858 discloses an aqueous disinfectant concentrate that contains an aldehyde, an alcohol with limited water miscibility, and preferably a nonionic surfactant. It is desired to provide formulations with improved low-temperature performance.

### STATEMENT OF THE INVENTION:

In one aspect of the present invention, there is provided a disinfectant composition comprising
(a) 15% to 49.75% biocide, by weight based on the weight of said composition,
(b) 15% to 49.75% water, by weight based on the weight of said composition, and
(c) 0.5% to 60% soluble salt, by weight based on the weight of said composition.

### DETAILED DESCRIPTION:

As used herein, "alkyl" is a saturated hydrocarbon, which may be linear, branched, cyclic, or a combination thereof.

As used herein, "flash point" is the flash point as measured as follows. Flash points between 25°C and 70°C are measured by the Abel-Pensky closed flash point tester according to DIN 51755, and flash points above 70°C are measured by the open cup Cleveland method.

As used herein, the phrase "a ratio of X: 1 or lower" means a ratio that has the value of Y:1, where Y is less than or equal to X. The case where Y is zero is included unless stated otherwise.

The term "microbicide", "biocide", "preservative" or "antimicrobial compound" refers herein to a compound useful for killing, inhibiting the growth of, or controlling the growth of microorganisms. Biocides include bactericides, fungicides and algicides. The term "microorganism" includes, for example, fungi (such as yeast and mold), bacteria, and algae.

As used herein, a "glycol ether" is a compound with structure (I): 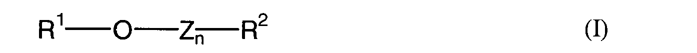 where n is equal to or greater than 1; n is less than 6; R¹ and R² are independently H or C₁ to C₄ alkyl; if n is 1, then at least one of R¹ and R² is not H; each unit -Z- is 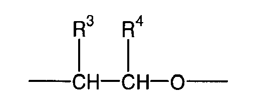 where, independently within each -Z- unit, independently hydrogen or methyl; and within each -Z- unit, R³ and R⁴ are not both methyl.

As used herein, a "Z1" unit is a -Z- unit in which R³ and R⁴ are both hydrogen, and a "Z2" unit is a -Z- unit in which either R³ or R⁴ is methyl.

As used herein, "ppm" means parts per million by weight.

As used herein, when it is stated that the composition of the present invention contains "little or no" of some ingredient, it is meant that either there is none of that ingredient in the composition or, if some of that ingredient is present, the amount of that ingredient is 100 ppm or less, based on the weight of the composition.

The composition of the present invention contains one or more biocide. Some suitable biocides include, for example, aldehydes, bromo-nitro compounds, and isothiazolones. Some suitable bromo-nitro compounds include, for example, dibromonitrilopropionamide ("DBNPA") and 2-bromo-2-nitropropane-1,3-diol ("bronopol").

In some embodiments, one or more biocide is used that is an aldehyde. In some embodiments, the biocide includes one or more of, for example, formaldehyde or succinic dialdehyde or glutaraldehyde. In some embodiments, glutaraldehyde is used. In some embodiments, no compound in the composition is a biocide other than glutaraldehyde.

In some embodiments, the composition of the present invention includes one or more biocide that is an aldehyde and also includes one or more biocide that is not an aldehyde. Suitable biocides that are not aldehydes include, for example, DBNPA, bronopol, quaternary ammonium biocides (including, for example, alkyl dimethyl benzyl ammonium chlorides, dialkyl dimethyl ammonium chlorides, tetrakishydroxymethyl phosphonium sulfate, tributyl tetradecyl phosphonium chloride, and other quaternary biocides), other biocides that are compatible with aldehyde biocides, and mixtures thereof.

Mixtures of suitable biocides are also suitable.

The amount of biocide in the composition of the present invention is 15% to 49.75% by weight, based on the weight of the composition. The amount of biocide is preferably 45% or less, by weight based on the weight of the composition.

The composition of the present invention contains one or more soluble salt. As used herein a salt is considered soluble if 2 grams of more of that salt can be dissolved at 25°C in 100 grams of a test composition made of equal parts by weight of biocide and water. The biocide in the test composition is the same biocide that will be used in the disinfectant composition. For some suitable salts, 10 grams of more of that salt can be dissolved in the test composition.

Each mole of salt that dissolves in the composition of the present invention produces at least one anion in solution and at least one cation in solution.

Suitable soluble salts include, for example, soluble salts that have cation of alkali metal or alkaline earth. In some embodiments, one or more salt is used that has cation of sodium, potassium, magnesium, or calcium.

Suitable soluble salts include, for example, soluble salts that have anion of halide, acetate, or nitrate. In some embodiments, one or more salt is used that has anion of chloride or acetate. In some embodiments, one or more salt is used that has anion that is not a halide ion. In some embodiments, one or more salt is used that has anion that is acetate.

It is contemplated that salts with anion other than halide have, in general, less tendency to promote corrosion of metals than salts with anion that is halide.

In some embodiments, one or more salt is used that is selected from magnesium chloride hexahydrate, calcium chloride hexahydrate, anhydrous magnesium chloride, anhydrous calcium chloride, potassium acetate, and mixtures thereof. In some embodiments, potassium acetate is used. In some embodiments, an anhydrous salt is used.

In embodiments in which one or more salt is used, the salt or salts may be mixed with the other ingredients of the composition by any method. When the salt is first mixed with one or more of the other ingredients of the composition, the form of the salt immediately prior to that mixing is herein called the form in which the salt is added to the composition.

In some embodiments, one or more salt is added to the composition in the form of a hydrated salt. In some embodiments, one or more salt is added to the composition in the form of an anhydrous salt. In some embodiments, every salt that is added to the composition is added in the form of an anhydrous salt.

Mixtures of suitable soluble salts are also suitable.

The total amount of all soluble salts in the composition of the present invention is 0.5% to 60% by weight based on the weight of the composition. In some embodiments, the amount of salt, by weight based on the weight of the composition, is preferably 30% or less; more preferably 12% or less, more preferably 7% or less. Independently, in some embodiments, the amount of salt, by weight based on the weight of the composition, is preferably 1% or more.

In some embodiments, there is little or no salt in the composition of the present invention that is not a soluble salt.

In some embodiments (herein called "solvent embodiments") the composition of the present invention contains one or more solvent. As used herein, a solvent is a compound that is not water and that is liquid at 25°C and one atmosphere pressure. Some suitable solvents contain one or more oxygen atoms per molecule.

In some solvent embodiments, the solvent in the composition of the present invention contains one or more glycol ether. Some suitable glycol ethers, for example, contain molecules in which each -Z- unit is a Z1 unit; molecules in which each -Z- unit is a Z2 unit; molecules that contain both one or more Z1 unit and one or more Z2 unit; or mixtures thereof. In some solvent embodiments, when the solvent as a whole is examined, the ratio of the weight of all Z1 units to the weight of all Z2 units is 0.66:1 or lower.

In some solvent embodiments, the solvent contains one or more glycol ether (herein called "GEA") that has structure (I) in which, within each -Z- unit, R³ and R⁴ are not both hydrogen. In some GEAs, every -Z- unit is the same as every other -Z-unit in that molecule. In some GEAs, one or more -Z- unit has R³ that is methyl and one or more -Z- unit has R⁴ that is methyl.

In some solvent embodiments, one or more GEA is used in which n is 2 or greater. In some solvent embodiments, one or more GEA is used in which n is 2 or 3. In some solvent embodiments, every GEA in the composition of the present invention has n of 2 or 3. In some solvent embodiments, one or more GEA is used in which n is 2. In some solvent embodiments, every GEA in the composition of the present invention has n of 2.

Some suitable GEAs include, for example, dipropylene glycol, dipropylene glycol monomethyl ether, propylene glycol methyl ether, tripropylene glycol monomethyl ether, propylene glycol n-propyl ether, dipropylene glycol n-propyl ether, dipropylene glycol n-butyl ether, propylene glycol n-butyl ether, dipropylene glycol dimethyl ether, and mixtures thereof.

In some solvent embodiments, the solvent contains one or more GEA in which n is 2 or 3 and also contains one or more GEA in which n is 1. In some of such embodiments, the solvent contains dipropylene glycol monomethyl ether and propylene glycol methyl ether.

In some solvent embodiments, every glycol ether in the composition is a GEA. In other solvent embodiments, the solvent contains one or more glycol ether that is not a GEA. In some solvent embodiments, for example, in addition to one or more GEA, the solvent also contains one or more "GEB," which is defined herein as a glycol ether of structure (I) in which, in every structure -Z-, both R³ and R⁴ are hydrogen. Some suitable GEBs include, for example, diethylene glycol, triethylene glycol, diglymes, and mixtures thereof. Diglymes are diethylene glycol dialkyl ethers, where the alkyl groups have 1 to 4 carbon atoms.

In some solvent embodiments in which a GEB is present, the ratio of the sum of the weights of all GEB compounds to the sum of the weights of all GEA compounds is 0.66:1 or lower.

It is considered herein that GEA compounds generally have lower human toxicity than GEB compounds.

In some solvent embodiments, one or more glycol ether is used that is a "GEAB," which is defined herein as a glycol ether having structure (I), where at least one -Z- unit is a Z1 unit and, in the same molecule, at least one -Z- unit is a Z2 unit. In some solvent embodiments in which a GEAB is used, the ratio of the weight of Z1 units within the molecule of that GEAB to the weight of all Z2 units within the same molecule is 0.66:1 or lower.

Mixtures of suitable glycol ethers are suitable.

In some solvent embodiments, one or more glycol ether is used that is water soluble. As used herein, a compound is water soluble if the amount of that compound that can be dissolved in 100 g of water at 25°C is 5 g or more. In some solvent embodiments, one or more glycol ether is used that is highly water soluble. As used herein, a compound is highly water soluble if the amount of that compound that can be dissolved in 100 g of water at 25°C is 50 g or more. In some solvent embodiments, one or more highly water soluble glycol ether is used that is miscible with water in all proportions. In some solvent embodiments, the entire solvent that is used is soluble in water. In some solvent embodiments, each ingredient in the solvent is water soluble. In some solvent embodiments, the entire solvent that is used is highly soluble in water. In some solvent embodiments, each ingredient in the solvent is highly water soluble.

In some solvent embodiments, the amount of solvent in the composition of the present invention is 0.1% to 70% by weight, based on the weight of the composition. In some solvent embodiments, the amount of solvent is, by weight, based on the weight of the composition, 1% or more; or 5% or more; or 25% or more; or 30% or more. Independently, in some solvent embodiments, the amount of solvent is, by weight, based on the weight of the composition, 65% or less.

In some embodiments of the present invention, no solvent is used in the disinfectant composition.

As used herein, a low diol is a compound with structure (III): 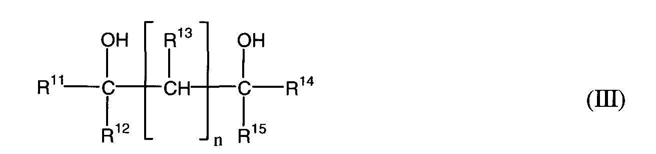 where n is 0, 1, or 2; each of R¹¹, R¹², R¹³, R¹⁴, R¹⁵ is independently hydrogen or any monovalent group. If n is 2, the two R¹³ groups may be the same or different. A compound is considered herein to be a low diol if it has structure (III), regardless of the nature of R¹¹, R¹², R¹³, R¹⁴, and R¹⁵. As used herein, an alkyl low diol is a low diol in which each of R¹¹, R¹², R¹³, R¹⁴, R¹⁵ is independently hydrogen or any monovalent alkyl group.

In some embodiments, the composition of the present invention contains little or no alkyl low diol. In some embodiments, the composition of the present invention contains no alkyl low diol. In some embodiments, the composition of the present invention contains little or no low diol. In some embodiments, the composition of the present invention contains no low diol.

In some embodiments, the composition of the present invention contains no surfactant. In other embodiments, the composition of the present invention contains one or more surfactant. Suitable surfactants may be nonionic, anionic, cationic, amphoteric, or a mixture thereof.

In some embodiments, the composition of the present invention contains little or no buffer. In some embodiments, the composition of the present invention contains no buffer.

Independently, in some embodiments, the composition of the present invention contains little or no organic lithium salts. In some embodiments, the composition of the present invention contains no organic lithium salts.

In some embodiments, the flash point of the composition of the present invention is equal to or higher than the flash point of acetone. Independently, in some embodiments, the flash point of the composition of the present invention is 55°C or higher.

In some solvent embodiments, each ingredient in the solvent of the composition of the present invention has flash point of 55°C or higher.

In some other solvent embodiments, one or more ingredient in the solvent has flash point of below 55°C. In such embodiments, when it is desired that the composition of the present invention have flash point of 55°C or higher, it is contemplated that the properties and the amount of each ingredient with flash point below 55°C are chosen so that the complete composition of the present invention will have flash point of 55°C or higher. Some suitable ingredients with flash points of below 55°C are, for example, C₁ to C₃ alkyl alcohols, such as, for example, isopropanol. Other examples are, for example, glycols or glycol ethers with flash points below 55°C, including, for example, propylene glycol methyl ether.

In some embodiments, no isopropanol is used. In some embodiments, no alcohol having flash point below 55°C is used. In some embodiments, no alcohol is used.

In some solvent embodiments, the amount of salt is chosen so that the ratio of the weight of all salt to the weight of all solvent is from 0.01:1 1 to 10:1. In some embodiments, the ratio of the weight of all salt to the weight of all solvent is 0.1:1 or higher, preferably 0.2:1 or higher. Independently, in some embodiments, the ratio of the weight of all salt to the weight of all solvent is 3:1 or lower, preferably 1:1 or lower.

In some embodiments, the sum of the weight of all salt plus the weight of all solvent will be 10% to 69% by weight, based on the weight of the composition.

The compositions of the present invention may be used in a variety of ways for a variety of purposes. For example, the composition of the present invention may be stored and used as a concentrate that may be added to water to provide the water solution with biocidal properties. Water with biocidal properties is useful, for example, in situations in which the water is in contact with metal (as in, for example pipes or tanks), because without biocidal properties, the water may encourage microbially induced corrosion in the metal. For example the removal of oil from under ground is sometimes enhanced by a waterflood, and the pipes, tanks, etc. that handle the water is prone to microbially induced corrosion. Many oilfields are in locations where the winter temperatures are relatively low. Despite the low temperatures, it is desirable to store the biocide concentrate outdoors and then pour it into a larger container, and to do some or all of these operations outdoors at relatively low temperatures.

In some situations, it is desirable to lower the cost of producing methyl vinyl ether by increasing raw material yield and decreasing energy intensity for producing the key intermediate dimethyl acetal (also called dimethyl acetal of acetaldehyde; DMA). This problem can be addressed by producing DMA via the combination of reactive distillation coupled with waste removal (by-product and impurity management) from the distillation tower along with recycling of process streams in order to recover raw materials thereby maximizing raw material yields. This can be done by a process for the production of DMA utilizing reactive distillation wherein by-products and impurities within the process are managed by continuous removal via a side-draw in the reactive distillation column. The catalyst required in the reactive distillation could be heterogeneous or homogeneous. The reactive distillation with side-draw allows efficient recycling process streams within one distillation column versus several separate distillation columns. Reducing the number of distillation columns helps to minimize overall energy use by reducing the energy required to both vaporize and condense the processed materials.

The combination of reactive distillation to achieve high raw material yields coupled with the recycling of material from subsequent processing steps made possible by an optimized side-draw on the reactive distillation column to remove impurities/by-products makes this process surprisingly less energy intensive and more efficient.

The use of side-draws within the reactive distillation column prevents impurities and by-products from accumulating within the column which might otherwise do so in a standard distillation column. For a given process and equipment, the locations of the side-draws and means to control the rate of removal from each is optimized based on raw material yields and process operability. Optimization is based primarily on the amount of valuable raw materials lost to the side streams and primarily the reflux ratio used to control the reactive distillation column. Raw materials include acetaldehyde, methanol, DMA and if employed, the heterogeneous catalyst. Whether this side-draw is removed as a vapor or liquid from that location is primarily dependent upon the type of catalyst used for the reactive distillation and the materials of construction of the side draw system. If homogeneous catalyst is used then a vapor side draw may preferable if the catalyst is non-volatile and therefore will not be present in the vapor being removed. This ensures efficient use of catalyst and mitigates concerns over the presence of the catalyst in further side-draw processing if desired.

Additionally, recycle of material from subsequent processing of the DMA intermediate back into the reactive distillation tower is extremely economically advantageous. The return locations of each recycle stream must also been optimized based upon their composition, energy input, and the physical constraints of the distillation column being used, i.e. the number of trays present and their operating efficiency. The locations of introducing recycle streams are important to the optimization of tower performance.

The side-draws could be eliminated by allowing impurities and by-products to exit with the distillation column tails stream or overhead stream. These streams could then be further treated in separate distillation towers to remove the impurities organics from the desired material. Such technology was practiced for several decades prior to implementing the technology disclosed herein. Similarly the recycle streams can also be treated by distillation or other separation technology to recover valuable components. Again, this was the technology used several decades prior to this technology. The historical process used significantly more energy to perform the desired separations. Their elimination, by incorporating all functions within one tower which serves as reactor, waste stream stripper, and recycler of process streams provides the significant reduction in energy use and increase in raw material efficiency.

### EXAMPLES

In the following Examples these abbreviations are used:

| | |
|---|---|
| Ucarcide™ 50 antimicrobial | 50% GA and 50% water by weight, based on the weight of Ucarcide™ 50 biocide |
| Ucarcide™ 42 antimicrobial | 42.5% GA, 7.5% alkyl dimethyl benzyl ammonium chloride and 58% water by weight, based on the weight of Ucarcide™ 42 biocide |
| anhy | anhydrous form of a salt |
| BDGA | Dowanol™ BDGA solvent (butyl diethylene glycol acetate) |
| DE | Dowanol™ DE solvent (diethylene glycol monethyl ether) |
| DEG | diethylene glycol |
| Diglyme | diethylene glycol dimethyl ether |
| DM | Dowanol™ DM solvent (diethylene glycol monomethyl ether) |
| DMM | Proglyde™ DMM solvent (dipropylene glycol dimethyl ether) |
| DPG | dipropylene glycol |
| DPM | Dowanol™ DPM solvent (dipropylene glycol monomethyl ether) |
| EPh | Dowanol™ EPh solvent (ethylene glycol phenyl ether) |
| GA | glutaraldehyde |
| i-PrOH | isopropanol |
| KOAc | potassium acetate |
| MPEG350 | methyl ether of HO-(CH₂CH₂O)₇-H |
| PG | propylene glycol |
| PGDA | Dowanol™ PGDA solvent (propylene glycol diacetate) |
| PM | Dowanol™ PM solvent (propylene glycol methyl ether) |
| PnB | Dowanol™ PnB solvent (propylene glycol n-butyl ether) |
| PnP | Dowanol™ Pnp solvent (propylene glycol n-propyl ether) |
| TMG | Dowanol™ TM solvent (trimethylene glycol) |

### Examples 1, C2, 3-7, C8, 9, C10, and C11

Formulations were prepared and tested as follows. Each of the compositions shown in Table 5 below was mixed and placed into a 1.2 milliliter tube and shaken. Approximately 1 milligram of copper sulfide powder was added as a nucleating agent. Samples were held at -50°C for at least 24 hours. The samples were then observed visually to detect phase separation. Five replicate samples were made and tested for each formulation shown. The samples shown in the table below were stable; that is, each sample showed no phase separation. Comparative formulations have Example Number that begins with "C."

Percentages are by weight, based on the weight of the formulation.

**Table 1: Formulations Stable at -50°C**

| | | | | Additive | % of |
|---|---|---|---|---|---|
| Example | Additive 1 | Additive 2 | ratio⁽⁹⁾ | %⁽¹⁰⁾ | Ucarcide™ 42 |
| 1 | MgCl₂, anhy | -- | -- | 11 | 89.0 |
| C2 | methanol | -- | -- | 26.6 | 73.4 |
| 3 | MgCl₂6H₂O | -- | -- | 32.1 | 67.9 |
| 4 | CaCl₂6H₂O | -- | -- | 33.3 | 66.7 |
| 5 | CaCl₂ anhy | DPM | 0.47:1 | 34.5 | 65.5 |
| 6 | CaCl₂ anhy | DPM | 0.24:1 | 39.4 | 60.6 |
| 7 | CaCl₂6H₂O | DPM | 0.36:1 | 44.8 | 55.2 |
| C8 | PM | none | -- | 45.0 | 55.0 |
| 9 | CaCl₂6H₂O | DPM | 0.72:1 | 48.7 | 51.3 |
| C10 | DMM | none | -- | 54.9 | 45.1 |
| C11 | DPM | none | -- | 57.3 | 42.7 |

| | | | | | |
|---|---|---|---|---|---|
| Note (9): weight ratio of Additive 1 to Additive 2. Note (10): The amount of the sum of Additive 1 plus Additive 1, by weight, based on the weight of the formulation. | | | | | |

### Examples 12-17, C18, 19-24, C25, C26, and 27

Samples were made and tested as in Examples and Comparative Examples 1-11 except that the test temperature was -45°C and that, instead of Ucarcide™ 42 antimicrobial, Glut50 (a solution of 50% GA and 50% water by weight, based on the weight of solution) was used. The formulations listed were all stable at -45°C.

**Table 2: Formulations Stable at -45°C**

| Example | | | | Additive | |
|---|---|---|---|---|---|
| No. | Additive 1 | Additive 2 | ratio ⁽⁹⁾ | %⁽¹⁰⁾ | % of Glut50 |
| 12 | MgCl₂ anhy | -- | -- | 10.5 | 89.5 |
| 13 | CaCl₂ anhy | -- | -- | 11.7 | 88.3 |
| 14 | MgCl₂ anhy | -- | -- | 32.1 | 67.9 |
| 15 | CaCl₂ anhy | DMM | 0.49:1 | 33.7 | 66.3 |
| 16 | CaCl₂ anhy | DPM | 0.47:1 | 34.7 | 65.4 |
| C17 | PM | -- | -- | 38.6 | 61.4 |
| 19 | CaCl₂ anhy | DMM | 0.24:1 | 38.6 | 61.4 |
| 20 | CaCl₂ anhy | DPM | 0.24:1 | 40.0 | 60.0 |
| 21 | CaCl₂ 6H₂O | DMM | 0.75:1 | 40.6 | 59.4 |
| 22 | CaCl₂ 6H₂O | DPM | 0.72:1 | 41.9 | 58.1 |
| 23 | CaCl₂ 6H₂O | DMM | 0.38:1 | 43.5 | 56.5 |
| 24 | CaCl₂ 6H₂O | DPM | 0.36:1 | 45.0 | 55.0 |
| C25 | DMM | -- | -- | 48.3 | 51.7 |
| C26 | DPM | -- | -- | 50.5 | 49.5 |
| 27 | MgNO₃6H₂O | -- | -- | 53.3 | 46.7 |

### Examples C28-C39, 40,C41, 42,C43, and C44

Samples were mixed and then cooled to -50°C. Samples that remained clear were labeled "pass," which those that showed phase separation were labeled "fail." Viscosity of the "pass" samples was assessed by a ball drop test. Using identical vials, a 7 gram sample of each formulation was placed in a vial, a metal ball of 2.8 mm diameter was placed on the surface, and the time for the ball to reach the bottom of the sample was recorded. The diameter of the vial was large compared to the diameter of the ball. Percent is by weight, based on the weight of the formulation. Comparative Examples have an example number that starts with "C." Results were as follows.

**Table 3: Freeze Stability and Viscosity at -50°C**

| Example | % of | % of | Additive | % of | | Drop Time |
|---|---|---|---|---|---|---|
| No. | Glut50 | DPM | Type | Additive | Stability | (min) |
| C28 | 50.2 | 37.4 | PnP | 12.5 | fail | |
| C29 | 50.0 | 25.1 | PnP | 25.0 | fail | |
| C30 | 49.8 | 37.6 | i-PrOH | 12.7 | fail | |
| C31 | 49.9 | 25.1 | i-PrOH | 25.0 | fail | |
| C32 | 49.9 | 37.5 | EPh | 12.6 | fail | |
| C33 | 49.9 | 25.0 | EPh | 25.0 | fail | |
| C34 | 50.0 | 25.0 | DMM | 25.0 | pass | 0.13 |
| C35 | 44.3 | 33.3 | PM | 22.4 | pass | 0.45 |
| C36 | 50.0 | 37.5 | DMM | 12.5 | pass | 0.55 |
| C37 | 57.1 | 28.4 | PM | 14.4 | pass | 1.19 |
| C38 | 50.0 | 50.0 | -- | 0 | pass | 2.03 |
| C39 | 50.0 | 37.5 | DPG | 12.5 | pass | 3.28 |
| 40 | 56.2 | 42.1 | MgCl₂ anhy | 1.7 | pass | 5.09 |
| C41 | 49.9 | 25.0 | DPG | 25.1 | pass | 7.58 |
| 42 | 64.1 | 32.0 | MgCl₂ anhy | 3.8 | pass | 11 |
| C43 | 50.0 | 37.4 | PM | 12.5 | pass | 0.53 |
| C44 | 50.0 | 25.0 | PM | 24.9 | pass | 0.29 |

### Examples C45 and 46-49

Examples were made and tested as Examples and Comparative Examples 28-44.

**Table 4: Freeze Stability and Viscosity at -50°C**

| Example | % of | % of | Additive | % of | | Drop Time |
|---|---|---|---|---|---|---|
| No. | Glut50 | diglyme | Type | Additive | Stability | (min) |
| C45 | 50.0 | 50.0 | -- | 0 | pass | 0.0 |
| 46 | 64.1 | 32.1 | MgCl₂ anhy | 3.9 | pass | 1.6 |
| 47 | 56.2 | 42.1 | MgCl₂ anhy | 1.7 | pass | 0.4 |
| 48 | 62.8 | 31.5 | KOAc | 5.7 | pass | 1.4 |
| 49 | 55.7 | 41.8 | KOAc | 2.5 | pass | 0.3 |
| 49A | 50.0 | 47.5 | KOAc | 2.5 | pass | |

### Examples C50 and 51-54

Examples were made and tested as Examples and Comparative Examples 45-49.

**Table 5: Freeze Stability and Viscosity at -50°C**

| Example | % of | % of | Additive | % of | | Drop Time |
|---|---|---|---|---|---|---|
| No. | Glut50 | PM | Type | Additive | Stability | (min) |
| C50 | 50.1 | 49.9 | -- | 0 | pass | 0.1 |
| 51 | 63.0 | 31.4 | KOAc | 5.7 | pass | 0.3 |
| 52 | 55.7 | 41.8 | KOAc | 2.5 | pass | 0.1 |
| 53 | 64.1 | 32.0 | MgCl₂ anhy | 3.9 | pass | 4.0 |
| 54 | 56.2 | 42.1 | MgCl₂ anhy | 1.7 | pass | 0.2 |

### Examples C38, C45, 49, 53, and 55: Steady-Shear Viscosity Testing

Samples were tested for steady-shear viscosity at -50°C using an Ares Rheometer with cone and cup geometry. The viscosity showed little or no dependence on shear rate in the range of 10 sec⁻¹ to 100sec⁻¹, and the viscosity reported below is the average viscosity over that range of shear rates. Viscosity is reported in Pascal*seconds (Pa*s), which is equivalent to 1,000 centipoise.

Example 55 is 50% by weight of a solution of equal parts by weight of glutaraldehyde and water; 43% by weight of diglyme; and 7% by weight isopropyl alcohol.

**Table 6: Steady-Shear Viscosity Test Results**

| Example No. | Temperature | Viscosity (Pa*s) |
|---|---|---|
| C38 | -50°C | 200 |
| C45 | -50°C | 4 |
| 53 | -50°C | 200 |
| C38 | -40°C | 14.2 |
| C45 | -40°C | 0.45 |
| 49A | -40°C | 0.89 |
| 55 | -40°C | 0.43 |

## Claims

1. A disinfectant composition comprising
(a) 15% to 49.75% biocide, by weight based on the weight of said composition,
(b) 15% to 49.75% water, by weight based on the weight of said composition, and
(c) 0.5% to 60% soluble salt, by weight based on the weight of said composition.

2. The composition of claim 1, wherein said composition further comprises 0.1% to 75% solvent, by weight based on the weight of said composition, wherein said solvent comprises one or more glycol ether having the structure (I): 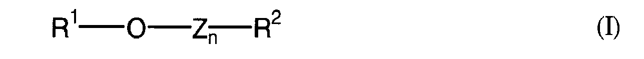 wherein n is equal to or greater than 1; wherein n is less than 6; wherein R¹ and R² are independently H or C₁ to C₄ alkyl; wherein, if n is 1, then at least one of R¹ and R² is not H; wherein each said unit -Z- is 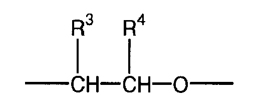 wherein, within each -Z- unit, R³ and R⁴ are each independently hydrogen or methyl;
wherein, within each -Z- unit, R³ and R⁴ are not both methyl; and
wherein the ratio of the weight of all the -Z- units in said solvent in which both of R³ and R⁴ are hydrogen to the weight of all the -Z- units in said solvent in which one of R³ and R⁴ is methyl is 0.66:1 or lower.

3. The composition of claim 2, wherein said solvent comprises one or more glycol ether (A), wherein each molecule of said glycol ether (A) has said structure (I) wherein, independently within each -Z- unit, one of R³ and R⁴ is hydrogen and the other of R³ and R⁴ is methyl.

4. The composition of claim 3, wherein said solvent comprises
(a) one or more glycol ether having said structure (A) wherein n is 2 or more, and
(b) one or more glycol ether having said structure (A) wherein n is 1.

5. The composition of claim 4 wherein said (b) is propylene glycol methyl ether.

6. The composition of claim 3, wherein said solvent additionally comprises one or more glycol ether (B) having structure (II): 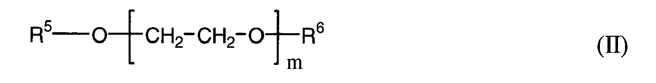 wherein m is equal to or greater than 1; wherein m is less than 6; wherein R⁵ and R⁶ are independently H or C₁ to C₄ alkyl; wherein, if n is 1, then at least one of R⁵ and R⁶ is not H;, and
wherein the ratio of the sum of the weights of all glycol ethers (B) to the sum of the weights of all glycol ethers (A) is 0.66:1 or less.

7. The composition of claim 1, wherein said biocide comprises a compound selected from the group consisting of glutaraldehyde, dibromonitrilopropionamide, and 2-bromo-2-nitropropane-1,3-diol.

8. The composition of claim 1, wherein said biocide comprises glutaraldehyde.

9. The composition of claim 1, wherein the amount of said soluble salt is 1% to 12% by weight, based on the weight of said composition.

10. The composition of claim 1, wherein one or more of said soluble salt is added to said disinfectant composition in the form of an anhydrous salt.
